# FASCICULE DE BREVET EUROPEEN

(11) **EP 2 144 518 B1**
(45) Date de publication et mention de la délivrance du brevet: **05.01.2011**
(21) Numéro de dépôt: 08788162.9
(22) Date de dépôt: 11.04.2008
(51) Int. Cl.: A41B 11/14, A41D 1/08, A41D 13/00

(54) **ARTICLE D'HABILLEMENT À EFFET DE CONTENTION HÉTÉROGÈNE POUR LA PRATIQUE D'UN SPORT**
KLEIDUNGSSTÜCK MIT HETEROGENEM KONKURRENZEFFEKT ZUM BETREIBEN EINER SPORTART
CLOTHING ITEM WITH HETEROGENEOUS CONTENTION EFFECT FOR PRACTISING A SPORT

(30) Priorité: 11.05.2007 FR 0755016
(43) Date de publication de la demande: 20.01.2010
(73) Titulaire: DECATHLON, 59650 Villeneuve d'Ascq (FR)
(72) Inventeur: BELLUYE, Nicolas, F-59777 Lille (FR); THEDON, Thibaud, F-34080 Montpellier (FR); PUCHAUX, Kostia, F-59000 Lille (FR)
(74) Mandataire: Cochonneau, Olivier
(86) Numéro de dépôt international: PCT/FR2008/050641
(87) Numéro de publication internationale: WO 2008/142334

(56) Documents cités:
- EP-A- 1 673 991
- WO-A-02/064073
- WO-A-2006/032096
- FR-A- 2 833 467
- JP-A- 2001 214 303
- US-A- 4 502 301

## Description

La présente invention est dans le domaine technique des articles d'habillement à effet de contention localisé, pour la pratique d'un sport, et plus particulièrement ceux obtenus par l'assemblage de premières pièces à élasticité de base et de secondes pièces à effet de contention.

La contention est bien connue dans le domaine médical, particulièrement s'agissant de chaussettes ou de bas de contention afin d'exercer un effet de compression différencié continu dont le but est thérapeutique puisqu'il s'agit d'améliorer la circulation sanguine notamment chez des patients souffrant d'insuffisance veineuse. Ces types d'articles textiles sont réalisés dans des matériaux élastiques tricotés, de préférence sur des métiers à tricoter circulaires. Ces articles sont généralement chauds, inesthétiques, et inappropriés pour la pratique d'un sport car ils génèrent des contraintes mécaniques sur les mouvements de l'usager.

Dans le domaine sportif, il est connu d'exercer un effet compressif en entourant d'une bande élastique soit une articulation en vue de la stabiliser, soit des masses musculaires en vue des les soutenir. Cette technique de compression est également appelée plus communément « strapping ».

Le document FR 2.879.900, au nom du demandeur, décrit un collant à effet de contention localisé pour la pratique d'un sport dans lequel les jambes sont animées d'un mouvement répétitif, notamment la course à pied et le vélo, principalement en vue du soutien des masses musculaires et du maintien de l'articulation du genou, sans générer d'efforts supplémentaires pour l'usager aux efforts nécessaires inhérents pour la pratique de la course à pied ou le vélo. Le collant décrit dans FR 2.879.900 ne permet pas d'améliorer le retour veineux et le drainage lymphatique significativement. De plus, cet article ne permet pas de maintenir le corps du coureur dans une position optimale en limitant les mouvements parasites.

Le document EP-A-1 673 991 décrit un collant à effet localisé de convention pour la pratique d'un sport comprenant des premieres pièces d'élasticité normale et des secondes pièces à effet de convention.

Il y a donc un besoin pour un article d'habillement adapté à la pratique d'une activité sportive donnée laquelle implique des mouvements particuliers et ce de façon répétitive permettant une réduction de la fatigue physiologique, physique et psychologique en optimisant à la fois la circulation sanguine, le drainage lymphatique, la proprioception, le soutien et le maintien des masses musculaires et des articulations, en corrigeant la posture, sans créer de contrainte mécanique susceptible d'être une source complémentaire d'effort aux mouvements que nécessite ladite activité sportive.

Afin de satisfaire aux besoins ci-dessus énoncés, la présente invention a pour objet un article d'habillement à effet de contention localisé, pour la pratique d'un sport, obtenu par l'assemblage de premières pièces à élasticité de base et de secondes pièces à effet de contention, et comprenant de manière caractéristique plusieurs types de secondes pièces aptes à produire des effets de contention différents, chaque type dans un intervalle de contention déterminé ; chacune desdites secondes pièces est agencée en sorte de produire un effet de contention soit longitudinale soit transversale ; lesdites secondes pièces des différents types sont agencées dans l'assemblage en sorte d'entourer totalement ou partiellement certaines sections transversales d'au moins un membre et/ou du torse et en sorte d'exercer un effet hétérogène de contention avec un gradient de pression décroissant vers le coeur.

Par secondes pièces à effet de contention longitudinale, on comprend que lesdites secondes pièces ont la même élasticité moyenne transversale que les premières pièces à élasticité de base, et qu'elles ont une élasticité moyenne longitudinale inférieure au moins de moitié à l'élasticité moyenne longitudinale des premières pièces.

Par contention transversale, on comprend que les secondes pièces ont la même élasticité moyenne longitudinale que les premières pièces à élasticité de base, les secondes pièces ayant une élasticité moyenne transversale inférieure au moins de moitié à l'élasticité moyenne transversale des premières pièces.

La notion d'élasticité moyenne prend en compte les variations normales de propriétés mécaniques dans toute fabrication textile, variations découlant des différences dues notamment aux provenances des matières premières, aux conditions de tissage ou tricotage, aux réglages des métiers. Les variations par rapport à une valeur moyenne peuvent être de l'ordre de 5 à 10 %, voire parfois plus.

Une seconde pièce selon la présente invention exerce un effet de contention longitudinale soit transversale et non les deux à la fois. En effet, un effet qui est soit de contention transversale et longitudinale aurait pour effet d'agir tel un garrot et de rendre très difficile voire impossible le passage de l'article d'habillement sur les membres de l'usager.

La contention transversale est exercée sensiblement dans la direction transverse du membre considéré ou du torse. La contention longitudinale est exercée sensiblement dans la direction longitudinale du membre considéré ou du torse.

Par membre, on comprend un membre inférieur : cuisse, jambe ou pied, ou un membre supérieur : bras, avant-bras et main.

Par effet hétérogène de contention avec un gradient de pression décroissant vers le coeur, on comprend que toutes les secondes pièces, ayant chacune un effet de contention dans un intervalle de contention donné, sont agencées dans l'assemblage de sorte que la suite des valeurs de contention appliquées par la succession desdites secondes pièces soit dégressive de l'extrémité distale du membre ou du tronc vers son extrémité proximale. Cet agencement des secondes pièces permet avantageusement d'augmenter le retour veineux et le drainage lymphatique.

L'article d'habillement, en fonction de l'activité sportive pratiquée, selon la présente invention présente avantageusement un effet de contention transversale pour le soutien d'une articulation, le maintien de masses musculaires, l'amélioration de la circulation sanguine, le drainage lymphatique et la proprioception et un effet de contention longitudinale pour guider les mouvements, réduire les mouvements parasites afin de maintenir le membre ou le torse sur lequel il est appliqué dans une position optimale.

L'usager n'est pas gêné dans la pratique d'une activité physique donnée de par les premières pièces à élasticité de base et les secondes pièces présentant une élasticité de base dans la direction longitudinale ou transversale selon les mouvements exécutés.

De plus, les secondes pièces exerçant des effets de contention selon des intervalles de contention différents, il est possible de renforcer le soutien et/ou le maintien des masses musculaires et des articulations et l'amélioration de la circulation sanguine, le drainage lymphatique et la proprioception selon les zones du corps considérées et les muscles utilisés afin d'optimiser ces différentes fonctions pour la pratique d'une activité sportive donnée.

Dans une variante, les secondes pièces sont choisies parmi trois types, dont les intervalles de contention correspondent à des effets de contention respectivement faibles, moyens et élevés.

Il est ainsi possible de moduler l'effet de contention transversale ou longitudinale selon les zones du corps particulièrement sollicitées lors d'une activité sportive donnée.

Dans une variante, le premier type à effet de contention faible correspond à un intervalle de pression de [10 ; 15] mm Hg, le deuxième type à effet de contention moyen à un intervalle de pression de [15 ;20] mm Hg et le troisième type à effet de contention élevé à un intervalle de pression de [20 ;25] mm Hg.

Les intervalles de contention sont déterminés par application des principes de la contention musculaire.

Dans une variante, l'article selon la présente invention se présente sous la forme d'une manche seule comprenant une partie d'avant-bras et une partie de bras.

Dans une sous-variante, la partie d'avant-bras comprend une seconde pièce à effet de contention transversale recouvrant au moins les muscles fléchisseurs des doigts. De préférence, ladite seconde pièce à effet de contention transversale exerce une pression sur les muscles fléchisseurs des doigts comprise dans un intervalle à effet de contention moyen, de préférence dans l'intervalle [15 ; 20] mmHg.

Dans une sous-variante, la partie de bras comprend une seconde pièce à effet de contention transversale recouvrant au moins les muscles biceps et triceps brachii. De préférence, ladite seconde pièce à effet de contention transversale exerce une pression sur les muscles biceps et triceps brachii comprise dans un intervalle à effet de contention faible, et de préférence dans l'intervalle [10 ; 15] mmHg.

La pression exercée transversalement sur les muscles fléchisseurs des doigts et les muscles biceps et triceps brachii permet d'augmenter le retour veineux et donc d'éviter les picotements et la sensation de bras lourds. Les picotements familièrement appelés « fourmillements » sont occasionnés lors du rétablissement d'une pression sanguine normale suite à une réduction du rythme de l'irrigation sanguine lorsqu'un ou plusieurs muscles ont été comprimés générant alors une surpression temporaire au niveau de l'étranglement. Ainsi, la contention transversale sur les muscles précités est particulièrement avantageuse lors de la pratique d'activités de montagne et notamment l'escalade où les muscles précités sont contractés et parfois comprimés, le bras étant souvent en extension et soutenant le poids du corps.

Par ailleurs selon l'altitude on observe une diminution de la pression partielle en dioxygène entre les artères et les veines, la pression atmosphérique diminuant, la pression dans le corps se détend et diminue, les tissus musculaires et les organes étant moins irrigués, le sujet a moins d'énergie. Ainsi la contention transversale exercée par lesdites secondes pièces pourrait permettre également de lutter contre ce phénomène lors d'activité en montagne de moyennes altitudes, notamment inférieures à 4.000 mètres.

Le dit article comprend également des premières pièces à élasticité de base, n'exerçant pas un effet de contention particulier puisque inférieur à 7 mmHg, et laissant l'usager parfaitement libre de ses mouvements.

Dans une sous-variante, l'article d'habillement comprend des éléments d'ancrage à ses extrémités et au niveau de l'articulation du coude.

De préférence, ces éléments d'ancrage sont disposés vers le poignet, le haut du bras et au niveau du coude, et sont des bandes élastiques permettant de maintenir en place la manche seule sur l'utilisateur en mouvement.

Dans une variante, l'article d'habillement selon la présente invention se présente sous la forme d'un maillot à manches longues comprenant une partie d'avant-bras, une partie de bras, une partie de poitrine, une partie d'épaule, une partie de dos et une partie d'abdomen.

Dans une sous-variante, la partie d'épaule comprend une première pièce à élasticité de base recouvrant le muscle deltoïde et la scapula.

L'article comprend deux parties d'épaules symétriques par rapport à l'axe longitudinal du torse. Ladite première pièce à élasticité de base n'oppose pas de contrainte mécanique supplémentaire, que ce soit dans la direction transversale ou longitudinale au torse, se traduisant par aucun effort supplémentaire pour l'usager. L'usager bénéficie ainsi d'une parfaite amplitude de mouvement au niveau de l'articulation de l'épaule. Cette disposition est particulièrement avantageuse pour la pratique de la natation et du surf.

Dans une sous-variante, la partie d'abdomen comprend une première pièce à élasticité de base recouvrant les muscles abdominaux.

Cette disposition permet de ne pas gêner la respiration de l'usager.

Dans une sous variante, la partie de poitrine comprend une seconde pièce à effet de contention transversale recouvrant les muscles pectoraux.

Les muscles pectoraux sont ainsi soutenus et maintenus, notamment lors de la natation ou du surf afin de favoriser l'action de propulsion du corps. Ladite seconde pièce permet également d'améliorer le retour veineux vers le coeur et notamment l'oxygénation des tissus musculaires.

Dans une sous-variante, la partie de dos comprend une seconde pièce à effet de contention transversale recouvrant les muscles du grand dorsal.

L'article d'habillement comprend deux parties de dos symétriques par rapport à l'axe longitudinal du torse. Ladite seconde pièce améliore le retour veineux et soutient le muscle du grand dorsal dans son action de propulsion notamment lors de la natation ou du surf. Ladite seconde pièce corrige également la posture en maintenant en extension le dos et en limitant les mouvements parasites.

De préférence, ladite seconde pièce à effet de contention transversale se prolonge sur le haut du dos par une seconde pièce à effet de contention longitudinale et se partage de part et d'autre de la nuque pour recouvrir notamment les muscles trapèzes et rhomboïdes.

De préférence, ladite seconde pièce recouvre les partie proéminentes des clavicules. La prolongation de la seconde pièce sur la nuque permet de maintenir en extension le cou et favorise l'action de propulsion lors de la nage. Dans une sous-variante, la partie d'abdomen comprend une seconde pièce à effet de contention transversale recouvrant le muscle grand oblique et le bas du muscle grand droit.

Ladite seconde pièce a notamment pour fonction de soutenir l'action stabilisatrice du tronc, atténuer l'inclinaison du buste dans le plan sagittal et les rotations pour maintenir une position optimale de nage.

Dans une sous-variante, les secondes pièces recouvrant les muscles pectoraux, du grand dorsal, du grand oblique, du grand droit, des trapèzes et rhomboïdes exercent une pression comprise dans un intervalle à effet de contention faible, et de préférence dans l'intervalle [10 ; 15] mmHg.

Dans une sous-variante, la partie d'avant-bras comprend une seconde pièce à effet de contention transversale recouvrant les muscles fléchisseurs des doigts exerçant une pression comprise dans un intervalle à effet de contention élevé, et de préférence dans l'intervalle [20 ; 25] mmHg.

Dans une sous-variante, la partie de bras comprend une seconde pièce à effet de contention transversale recouvrant les muscles biceps et triceps brachii exerçant une pression comprise dans un intervalle à effet de contention faible, et de préférence dans l'intervalle [10 ; 15] mmHg.

Les secondes pièces disposées sur la partie d'avant- bras et la partie de bras ont pour fonction d'améliorer le retour veineux, diminuer la sensation de picotements et de « bras lourds ».

Dans une sous-variante, la seconde pièce à effet de contention transversale sur la partie de dos comprend une fausse couture afin de plaquer ledit article sur le dos de l'usager, de préférence longeant la colonne vertébrale.

La fausse couture permet également de conserver malgré l'étendu de la seconde pièce une contention importante sur la nuque.

Dans une sous-variante, ledit article d'habillement comporte des troisièmes pièces destinées à recouvrir les aisselles se présentant sous la forme de filets (étoffe à mailles très ouvertes) pour favoriser l'évacuation de la chaleur et de la transpiration.

Les filets étant de préférence élastiques ne gênent pas l'usager dans ses mouvements.

Dans une variante, l'article d'habillement selon la présente invention se présente sous la forme d'un collant comprenant une partie antérieure de la cuisse, une partie postérieure de la cuisse, une partie de genou et une partie de jambe.

Dans une sous-variante, ladite partie antérieure de la cuisse comprend une seconde pièce à effet de contention transversale recouvrant le muscle droit, le muscle du vaste externe, les muscles adducteurs et/ou du vaste interne.

Pour la pratique de la natation, la seconde pièce recouvre de préférence les muscles adducteurs et le vaste interne afin d'augmenter le flux sanguin vers le coeur et diminuer les sensations de picotements et de « jambes lourdes ».

Pour la pratique du surf, le vaste interne est comprimé par ladite seconde pièce et non les muscles adducteurs afin d'augmenter le flux sanguin vers le coeur et améliorer l'échauffement et la récupération.

Dans une sous-variante, ladite partie postérieure de la cuisse comprend une seconde pièce à effet de contention transversale recouvrant les muscles biceps fémoral et les muscles adducteurs ou les muscles semi-membraneux.

Pour la pratique du surf, les muscles semi-membraneux sont comprimés et non les muscles adducteurs afin d'améliorer l'échauffement et la récupération.

Pour la pratique de la natation, les muscles adducteurs sont comprimés et non les muscles semi-membraneux, afin d'améliorer le retour veineux et diminuer les crampes et la sensation de picotements.

Dans une sous-variante, la partie de genou comprend une seconde pièce à effet de contention transversale recouvrant sur sa face antérieure la tête de la fibula et sur sa face postérieure l'intérieur de l'articulation du genou pour se prolonger sur la partie haute du gastrocnémien en passant sous ladite articulation du genou.

Cette seconde pièce est ainsi disposée en particulier pour la natation. Elle permet de soutenir et guider le travail de flexion et d'extension du genou lors de la propulsion du nageur.

De préférence, lesdites secondes pièces à effet de contention soit transversale soit longitudinale exercent une pression comprise dans un intervalle à effet de contention faible, et de préférence dans l'intervalle [10 ; 15] mmHg

Dans une sous-variante, ladite partie de jambe comprend une seconde pièce à effet de contention transversale recouvrant les muscles gastrocnémiens et les muscles extenseurs des orteils.

Lors de la pratique de la natation, cette disposition permet d'augmenter le flux sanguin vers le coeur et diminuer les sensations de crampes et de picotements. Pour la pratique du surf, on obtient davantage une amélioration de la récupération et de l'échauffement.

De préférence, ladite seconde pièce à effet de contention transversale exercent une pression comprise dans un intervalle à effet de contention élevé, et de préférence dans l'intervalle [20 ; 25] mmHg.

Dans une sous-variante, ledit article d'habillement comporte des troisièmes pièces, notamment à mailles très ouvertes, par exemple se présentant sous la forme de filet, destinées à recouvrir le creux poplité du genou pour favoriser l'évacuation de la chaleur et de la transpiration.

Dans une variante, l'article d'habillement selon la présente invention se présente sous la forme d'une combinaison à manches longues, notamment pour la natation, comprenant une partie d'avant-bras, une partie de bras, une partie de poitrine, une partie d'épaule, une partie de dos, une partie d'abdomen, une partie de fessier, une partie antérieure de la cuisse, une partie postérieure de la cuisse, une partie de genou et une partie de jambe selon l'une quelconque des variantes de réalisations décrites ci-dessus.

Dans une sous-variante, ladite partie de dos comprend une seconde pièce à effet de contention longitudinale recouvrant les muscles érecteurs du rachis et se prolongeant vers le bas du dos sur la partie basse du muscle du grand fessier et le muscle tenseur du fascia lata, et vers le haut du dos longeant la colonne vertébrale pour se partager de part et d'autre de la nuque et recouvrir notamment les muscles trapèzes et rhomboïdes. Ladite seconde pièce exerce une pression comprise dans un intervalle à effet de contention faible, et de préférence dans l'intervalle [10 ; 15] mmHg. Dans une variante, la partie d'abdomen comprend une seconde pièce à effet de contention longitudinale recouvrant, en passant sous le nombril et de part et d'autre des hanches, les muscles du grand oblique et du grand droit. Ladite seconde exerce une pression comprise dans un intervalle de contention faible, et de préférence dans l'intervalle [10 ; 15] mmHg.

Les muscles précités sont soutenus par ladite seconde pièce dans leur action stabilisatrice du buste. Ladite seconde pièce permet d'atténuer l'inclinaison du buste dans le plan sagittal et de maintenir une position optimale particulièrement pour la pratique de la natation. La description de l'agencement des premières, secondes et troisièmes pièces des parties d'avant-bras, de bras, de poitrine, d'épaule, de fessier, antérieure et postérieure de la cuisse, de genou et de jambe de la combinaison n'est pas reprise puisqu'elle correspond à l'agencement décrit notamment pour le maillot à manches longues et le collant pour la natation.

Dans une variante, l'article d'habillement comporte une première pièce à élasticité de base recouvrant l'articulation du coude, et préformée selon un angle de 45° correspondant à l'angle de flexion naturel du coude.

La présente invention sera mieux comprise à la lecture de la description des exemples de réalisation ci-après, cités à titre non limitatif, et illustrés dans les figures ci-après, annexées à la présente :
- les figures 1A et 1b sont respectivement des vues avant et de dos d'une combinaison pour la pratique de la natation ;
- les figures 2A et 2B sont respectivement des vues avant et de dos d'un premier exemple de maillot à manches longues pour la pratique de la natation ou du surf ;
- les figures 3A et 3B sont respectivement des vues avant et de dos d'un second exemple de maillot à manches longues pour la pratique du surf ou de l'escalade ;
- les figures 4A et 4B sont respectivement des vues avant et de dos d'un collant pour la pratique du surf ou la natation ;
- les figures 5A et 5B sont respectivement des vues avant et de dos d'une manche seule pour la pratique de l'escalade et/ou d'activités de montagne ;
- les figures 6 et 7 sont des représentations schématiques de l'appareil de mesure de pression du type KIKUHIME.

Les articles d'habillement selon la présente invention, notamment combinaison, maillot à manches longues et manche seule, sont constitués par l'assemblage de premières, secondes et troisièmes pièces selon une technique limitant ou éliminant les risques d'hétérogénéité d'élasticité entre les pièces assemblées, comme expliqué ci-après. Les bords des pièces assemblées sont juxtaposés, sans aucune superposition. Les pièces dénommées ci-après premières pièces sont coupées dans un matériau élastique, d'élasticité normale. Il s'agit par exemple d'un tricot indémaillable à armure dénommée charmeuse de l'ordre de 170 g/m², constitué de fils 80/20 polyamide-élasthane. Les pièces dénommées ci-après secondes pièces sont découpées dans un matériau textile élastique - éventuellement comportant un élastomère, par exemple du néoprène - dont l'élasticité moyenne dans le sens transversal ou longitudinal est nettement inférieure à celui des premières pièces dans un rapport d'au moins 2.

Les secondes pièces sont de préférence découpées dans un même matériau élastique. En effet, en réduisant plus ou moins la quantité de matière formant les secondes pièces dans l'article d'habillement d'après la taille à laquelle l'article doit correspondre, les secondes pièces exercent, une fois ledit article porté, un effet de contention faible, moyen, ou fort.

Pour la mesure de l'élasticité moyenne, les allongements sont calculés sous une force de 15N. Dans les exemples précités, les élasticités moyennes des premières et secondes pièces dans le sens longitudinal ou transversal selon le type de contention recherché, sont quasiment identiques, avec un écart inférieur à 5%, tandis que l'élasticité moyenne entre les premières et secondes pièces, dans le sens longitudinal ou transversal, est dans un rapport au minimum de 2.

Les pièces dénommées ci-après troisièmes pièces, sont des pièces dont l'élasticité moyenne est supérieure à celle de la première pièce. Il s'agit notamment d'un filet à mailles relativement ouvertes, de l'ordre de quelques millimètres, permettant d'assurer également un effet d'aération dans les zones qu'il couvre.

L'assemblage se fait par toute technique conventionnelle, notamment par couture, collage ou thermosoudage. S'agissant d'un assemblage coupé/cousu les coutures sont de préférence du type flatlock ou surjet, en utilisant des fils mousses, par exemple texturés, au trameur. Dans ce cas, il est souhaitable de régler la tension des fils des machines à coudre à une valeur inférieure à celle couramment utilisée afin de limiter l'effet négatif que pourrait présenter des coutures sur l'homogénéité des élasticités moyennes des pièces assemblées.

Par ailleurs, le nombre de panneaux mis en oeuvre dans la confection peut varier, s'agissant notamment d'un article d'habillement pour homme ou pour femme, et ce qui importe ce n'est pas le nombre de panneaux mais bien la répartition des premières pièces, des différentes types de secondes pièces et des troisièmes pièces sur les parties du corps concernées dudit article.

Sur les figures, les panneaux formant les premiers pièces à élasticité de base sont représentés sans hachure, les panneaux formant les secondes pièces à effet de contention transversale sont représentés avec des hachures transversales, les panneaux formant les secondes pièces à effet de contention longitudinale sont représentés avec des hachures longitudinales, et les panneaux formant les troisièmes pièces avec des hachures obliques.

Dans la suite de la description, il sera fait usage du terme panneau dans la description précise des modes de réalisation illustrés dans les figures et des termes premières, secondes et troisièmes pièces pour ce qui concerne leur disposition localisée sur les zones du corps.

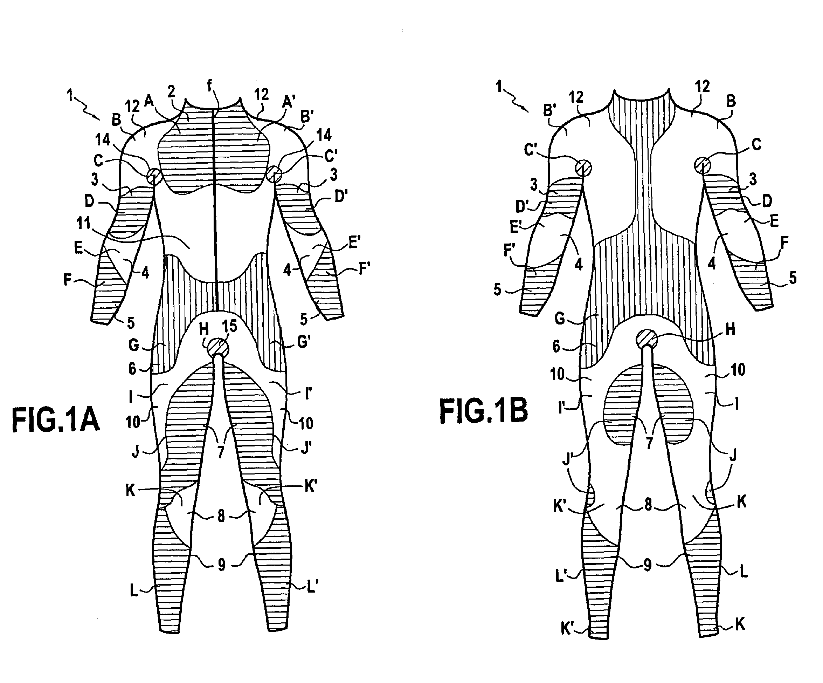

La combinaison 1, représentée aux figures 1A et 1B, est particulièrement adaptée pour la pratique de la natation. Elle est formée par l'assemblage de vingt-deux ou vingt-trois panneaux consistant en huit panneaux ayant la structure des premières pièces à élasticité de base [B, B', E, E', I, I', K, K'], dix panneaux ayant la structure des secondes pièces à effet de contention transversale [A, A', D, D', F, F', J, J', L, L'], un panneau ayant la structure des secondes pièces à effet de contention longitudinale [G], et deux [ou trois] panneaux ayant la structure des troisièmes pièces C, C', [et éventuellement H], formées d'un filet à mailles ouvertes. Les panneaux [A, A', D, D', F, F', J, J', L, L'], ont une élasticité moyenne transversale inférieure à celle des panneaux [B, B', E, E', I, I', K, K'], et le panneau [G] a une élasticité moyenne longitudinale inférieure à celle des panneaux [B, B', E, E', I, I', K, K'].

Au niveau de la partie supérieure de la combinaison 1, à la figure 1A, une seconde pièce 2 à effet de contention transversale recouvre le muscle grand pectoral. La seconde pièce 2 est formée de deux panneaux A et A' symétriques par rapport à une fermeture à glissière f, permettant l'enfilage de la combinaison et qui descend jusqu'au niveau du nombril. Le muscle grand pectoral permet de croiser, lever et baisser les bras devant le thorax et ce éventuellement en rotation. La seconde pièce 2 a pour fonction de maintenir et soutenir le muscle du grand pectoral dans son action de propulsion du corps lors de la nage, et également d'augmenter le retour veineux.

La seconde pièce 3, formée des panneaux D et D', recouvre les muscles biceps et triceps (longue portion, vaste externe, vaste interne) brachii.

La seconde pièce 5, formée des panneaux F et F', recouvre les muscles fléchisseurs des doigts. Les muscles biceps et triceps brachii agissent de façon coordonnée pour lever et abaisser l'avant-bras. La seconde pièce 3 s'étend sur la partie postérieure au dessus du coude jusqu'au niveau de l'aisselle, et encercle totalement la partie antérieure du bras en évitant le pli du coude. Les prolongements des muscles triceps et biceps brachii sur l'omoplate ne sont pas couverts par la seconde pièce 3 afin de ne pas gêner les mouvements de l'épaule. Le triceps brachial est un extenseur du coude, il contrôle l'élévation et l'abaissement de l'avant-bras en synergie avant le biceps brachial.

Les secondes pièces 3 et 5 exercent un effet de contention transversale afin de favoriser le retour veineux, diminuer les picotements et la sensation de « bras lourds ».

A la figure 1B, la seconde pièce 6 à effet de contention longitudinale, formée du panneau G, recouvre la partie inférieure du muscle grand dorsal jusqu'au dessus des fessiers. La seconde pièce 6 ne recouvre pas les clavicules gauche et droite. Le muscle grand dorsal est un muscle plat très étendu, couvrant la moitié inférieure du dos. Il mobilise les bras et est particulièrement utilisé lors de la nage. La seconde pièce 6 soutient l'action de propulsion du corps par le grand dorsal, elle participe ainsi au maintien en extension du dos et favorise le retour veineux.

La seconde pièce 6 se prolonge du dos vers l'abdomen et recouvre les muscles grands obliques, le bas du grand droit, les érecteurs du rachis et se prolonge vers le bas du grand fessier et du tenseur du fascia lata. Cette seconde pièce 6 est ici formée d'un seul panneau G encerclant totalement le bassin du nageur. Le muscle grand droit sert à fléchir le buste vers l'avant et permet en outre de tendre la paroi abdominale et comprimer les viscères. Le grand oblique est un muscle large et superficiel qui s'étend latéralement et sur l'avant du tronc. Il se divise en deux chefs : une portion thoracique supérieure et une portion latérale inférieure, lesquelles portions se rejoignent latéralement sur le bassin. Le grand oblique sert à fléchir le tronc en avant et à tourner le buste d'un côté ou de l'autre. Le grand fessier s'étend vers le bas et l'extérieur en enveloppant la protubérance latérale et postérieure du bassin. Le grand fessier est un extenseur de la cuisse au niveau de l'articulation de la hanche et permet de la tirer vers l'arrière pendant les battements de jambes lors de la nage. Le tenseur du fascia lata est un petit muscle allongé qui s'étend de l'os iliaque au grand trochanter. Il permet la rotation interne du fémur, et est également fléchisseur abducteur de la cuisse sur le bassin. La seconde pièce 6, de par son effet de contention longitudinale, soutient l'action des muscles qu'elle recouvre dans leur fonction de stabilisation du tronc, et atténue l'inclinaison du buste vers l'avant afin de maintenir une position optimale lors de la nage.

Les premières pièces à élasticité de base 11 et 12 recouvrent respectivement les muscles abdominaux et le muscle deltoïde et la scapula (l'omoplate). La première pièce 12 n'oppose pas de résistance mécanique aux mouvements des bras du nageur et permet une grande amplitude articulaire de l'épaule. La première pièce 11 laisse les mouvements de la respiration abdominale libres. Les premières pièces 11 et 12 sont dans cet exemple 1 formées par les panneaux B et B'.

La seconde pièce 7 à effet de contention transversale recouvre sur la face antérieure de la cuisse le muscle droit antérieur, le vaste externe, le vaste interne et les adducteurs. La seconde pièce 7 se prolonge vers l'intérieur et le haut de la partie postérieure de la cuisse, vers l'extérieur et le bas pour se terminer sur le côté de l'articulation du genou, et vers l'intérieur et le bas pour couvrir l'articulation du genou (patte d'oie). Les muscles précités sur la face postérieure de la cuisse ont une action commune d'extenseur du genou. Sur la face postérieure de la cuisse, la seconde pièce 7 recouvre le muscle biceps fémoral et les adducteurs. La seconde pièce 7 est formée de deux panneaux J et J', et a pour fonction d'améliorer le retour veineux, diminuer les sensations de picotements et de « jambes lourdes » ainsi que les crampes.

Au niveau du genou, la seconde pièce 7 passe sur l'extérieur de celui-ci et se termine sur la tête de la fibula. La seconde pièce 7 passe également sur l'intérieur de l'articulation du genou (patte d'oie) et se prolonge sur la partie postérieure en passant sous le creux de l'articulation du genou et couvre la partie haute des muscles gastrocnémiens (les muscles jumeaux de la jambe). Cette dernière disposition de la seconde pièce 7 permet de soutenir et guider le travail de flexion et d'extension du genou lors de la propulsion du nageur.

La seconde pièce 9 à effet de contention transversale recouvre les muscles gastrocnémiens et les muscles extenseurs des orteils afin d'augmenter le retour veineux, diminuer la sensation de picotements et les crampes. La seconde pièce 9 est formée des deux panneaux L et L'.

La première pièce à élasticité de base 4, formée par les deux panneaux E et E', recouvre l'articulation du coude et le creux poplité du coude. Les deux panneaux E et E' sont dans une matière élastique de base préformée selon un angle de 45° correspondant à l'angle naturel de flexion du coude. Aucune contrainte mécanique n'est ainsi exercée sur l'articulation du coude et ne gêne les mouvements de l'avant-bras.

Une autre première pièce à élasticité de base 10, formée des panneaux I et I', recouvre partiellement les parties postérieure et antérieure de la cuisse et notamment la bandelette de Maissiat et se prolonge vers la bordure inférieure de la seconde pièce 6.

Une première pièce à élasticité de base 8, formée des panneaux K et K', recouvre sur la face antérieure de la jambe, la patella (ou rotule) et sa périphérie supérieure et inférieure. La seconde pièce 9 se prolonge vers le pied en recouvrant notamment le muscle jambier extérieur et le tibia.

Les troisièmes pièces 14, sous forme de filet à mailles ouvertes, formées des panneaux C et C' recouvrent les aisselles pour évacuer la chaleur et la transpiration. Eventuellement une troisième pièce 15 formée du panneau H recouvre le périnée.

Les premières pièces à élasticité de base 4, 8, 10, 11, 12 ont dans cet exemple 1 un effet de contention inférieure à 7 mmHg.

La seconde pièce 5 exerce un effet de contention fort, de préférence compris dans l'intervalle [20 ; 25] mmHg. Les secondes pièces 3 et 2 exercent un effet de contention faible, de préférence compris dans l'intervalle [10 ; 15] mmHg. Ainsi, les secondes pièces 2, 3 et 5 exercent un effet discontinu de contention avec un gradient de pression décroissant de l'extrémité distale du membre, ici l'avant-bras, vers le coeur.

De même, la seconde pièce 9 exerce un effet de contention fort, de préférence compris dans l'intervalle [20 ; 25] mmHg. Les secondes pièces 6 et 7 exercent un effet de contention faible, de préférence compris dans l'intervalle [10 ; 15] mmHg.

L'augmentation du retour veineux est ainsi parfaitement assurée et permet d'améliorer l'oxygénation des tissus et diminuer les sensations de picotements et de « membres lourds ».

La combinaison 1 en comprimant plus ou moins localement le corps, et selon un sens longitudinal ou transversal, améliore la circulation sanguine, le drainage lymphatique et la proprioception. Elle maintient les masses musculaires et stabilise les articulations pour prévenir des traumatismes, guide les mouvements, réduit les mouvements parasites, et maintient une position hydrodynamique dans l'eau.

Dans la description des premières, secondes et troisièmes pièces ci-après, il ne sera plus fait mention des panneaux proprement dits, qui sont du ressort du montage de l'article d'habillement lors de sa confection, mais uniquement des premières, secondes et troisièmes pièces, caractéristiques de la présente invention.

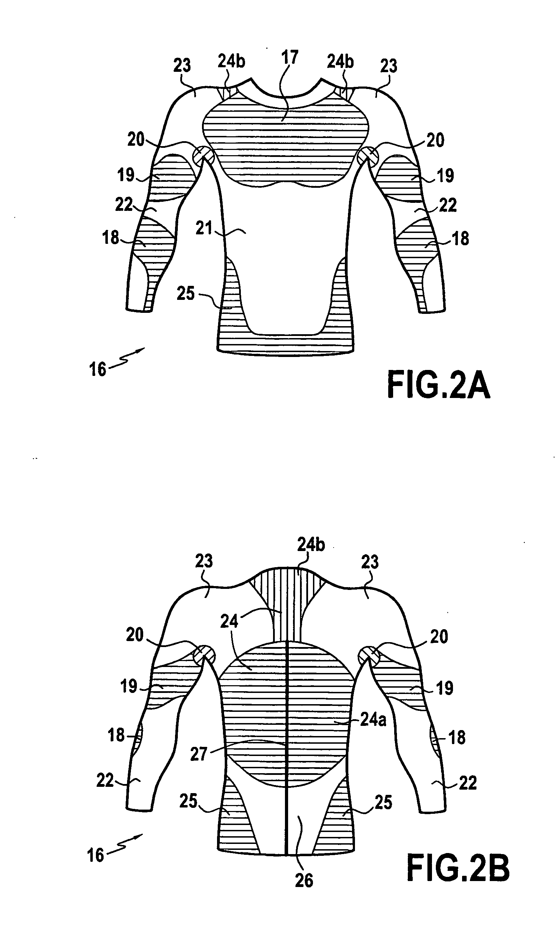

Dans le second exemple qui est illustré aux figures 2A et 2B, le maillot à manches longues 16 est particulièrement destiné à la pratique de la natation et du surf. Ce maillot 16 présente des différences avec l'agencement des premières, secondes et troisièmes pièces disposées sur le haut du corps de la combinaison 1 pour la natation.

La description qui va suivre s'attache à souligner ces différences.

Les secondes pièces 17,18 et 19 sont équivalentes respectivement aux secondes pièces 2, 3 et 5 sur la combinaison 1. Les premières pièces 21, 22 et 23 sont équivalentes respectivement aux premières pièces 11, 4 et 12 sur la combinaison 1. De même, la troisième pièce 20 est équivalente à la troisième pièce 14 sur la combinaison 1.

La seconde pièce 24 est en deux parties, une première partie 24a à effet de contention transversale qui recouvre le muscle du grand dorsal et qui se prolonge sur le haut du dos par une seconde partie 24b à effet de contention longitudinale qui se partage de part et d'autre de la nuque pour recouvrir notamment les muscles trapèzes et rhomboïdes. La seconde pièce 24 a pour fonctions d'améliorer le retour veineux, soutenir le grand dorsal dans son action de propulsion du corps, notamment lors de la nage, et favoriser le maintien en extension du cou. La seconde pièce 24 est divisée en deux parties sensiblement symétriques par une fausse couture 27 partant du bord supérieur de la seconde pièce 24a descendant jusqu'aux fessiers en longeant la colonne vertébrale. La fausse couture 27 a pour but de maintenir la seconde pièce 24b et la première pièce 26 plaquées contre le dos.

La seconde pièce 25 à effet de contention transversale, recouvre sur l'abdomen les muscles du grand oblique et le bas du grand droit. Elle a pour fonction d'augmenter le retour veineux, soutenir lesdits muscles dans leur action stabilisatrice du tronc et atténuer l'inclinaison du buste dans le plan sagittal afin de maintenir une position optimale, notamment lors de la nage.

La seconde pièce 18 exerce un effet de contention fort, de préférence compris dans l'intervalle [20 ; 25] mmHg. Les secondes pièces 17, 19 et 24 exerce un effet de contention faible, de préférence compris dans l'intervalle [10 ; 15] mmHg. La pression exercée est donc dégressive d'une extrémité distale, c'est à dire du poignet, vers le proximal à savoir le coeur.

Le maillot 16 maintient le buste, les épaules et la nuque dans une position idéale pour faciliter la respiration et favoriser l'action propulsive du mouvement des bras. Il permet également, comme la combinaison 1, d'améliorer la circulation sanguine, le drainage lymphatique et la proprioception. En maintenant les masses musculaires, les articulations sont stabilisées et le risque de traumatisme est diminué.

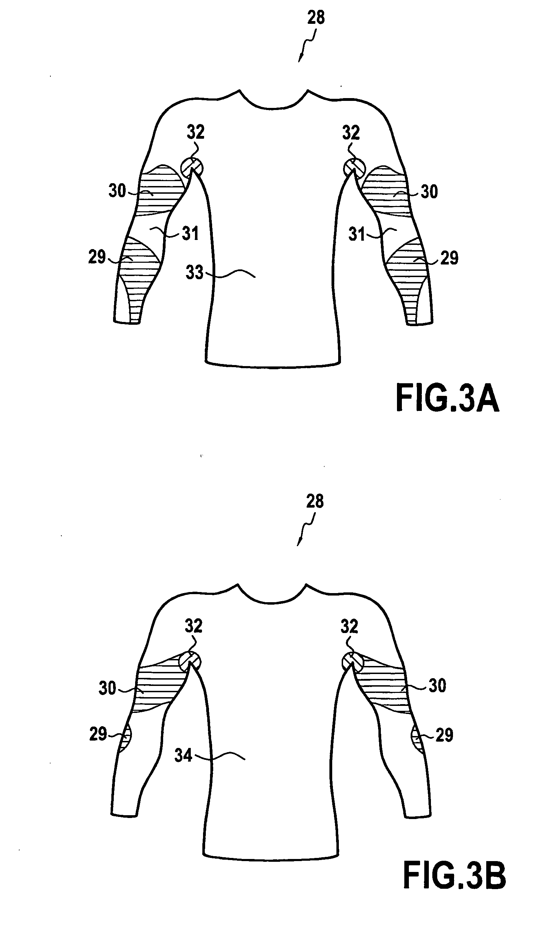

Le maillot à manches longues 28, représenté aux figures 3A et 3B, est particulièrement adapté pour la pratique du surf ou de l'escalade.

Les secondes pièces 29 et 30 sont équivalentes respectivement aux secondes pièces 5 et 3 de la combinaison 1, et permettent en outre en exerçant en effet de contention fort, de préférence compris dans l'intervalle [20 ; 25] mmHg, d'augmenter le retour veineux pour améliorer l'échauffement et la récupération d'une activité physique.

La troisième pièce 32 est équivalente à la troisième pièce 14 de la combinaison 1. La première pièce 31 est équivalente à la première pièce 4 de la combinaison 1. Le maillot 28 comprend ici deux premières pièces à élasticité de base 33 et 34 couvrant respectivement les faces antérieure et postérieure du torse. Les premières pièces 31, 33 et 34 exercent un effet de contention très faible, de préférence inférieure à 5 mmHg afin de ne pas gêner l'amplitude des mouvements du surfeur.

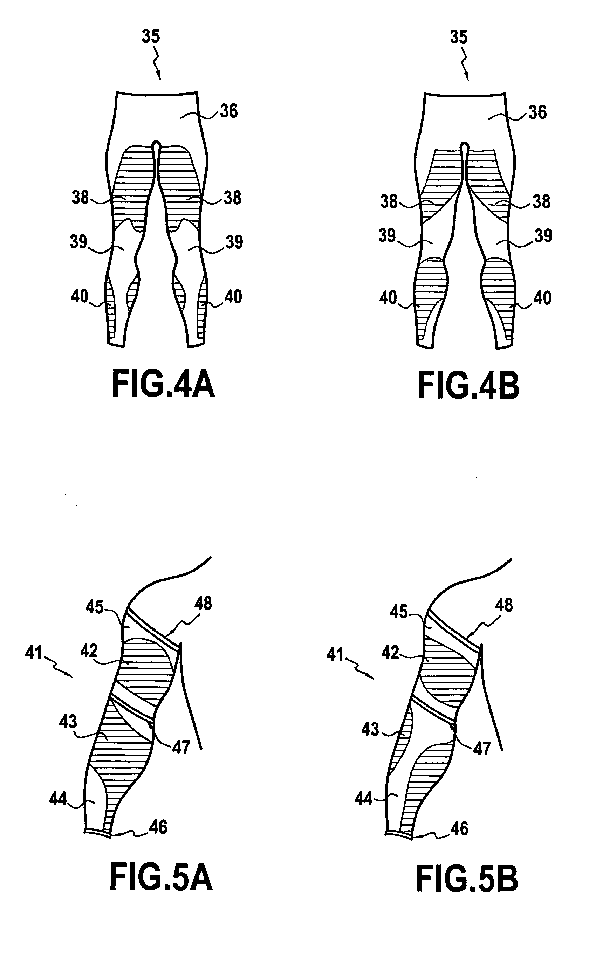

Le collant 38, représenté aux figures 4A et 4B, est particulièrement destiné à la pratique du surf ou de la natation, il peut être combiné avec les maillots 16 et 28 décrits précédemment.

La seconde pièce 40 est équivalente à la seconde pièce 9 sur la combinaison 1. La première pièce à élasticité de base 39 recouvre notamment la tête de la fibula et le creux poplité du genou afin de laisser une grande amplitude articulaire. La première pièce à élasticité de base 36 recouvre les fessiers et la bande de Maissiat. La seconde pièce 38 à effet de contention transversale s'étend jusqu'au sillon sous-fessier en recouvrant sur la face antérieure de la cuisse : le vaste externe, le droit antérieur et le vaste interne, et sur la face postérieure : les muscles biceps fémoral et semi-membraneux.

La seconde pièce 40 exerce un effet de contention transversale fort, de préférence compris dans l'intervalle [20 ; 25] mmHg. La seconde pièce 38 exerce un effet de contention transversale moyen, de préférence compris dans l'intervalle [10 ; 15] mmHg.

Les premières pièces à élasticité de base 36 et 39 exercent un effet de contention très faible, de préférence inférieure à 5 mmHg. Le collant 35 est adapté pour améliorer la récupération et l'échauffement lors d'activités physiques en augmentant le retour veineux.

Le manchon 41, représenté aux figures 5A et 5B est particulièrement destiné à la pratique de l'escalade et/ou aux activités en montagne.

Les secondes pièces 42 et 43 à effet de contention transversale sont équivalentes respectivement aux secondes pièces 3 et 5 sur la combinaison 1 à la différence que la seconde pièce 43 exerce un effet moyen de contention, de préférence compris dans l'intervalle [15,20] mmHg. Les premières pièces à élasticité de base 44 et 45 sont préformées selon un angle de 45° correspondant à l'angle de flexion naturel du coude. De plus, les premières pièces 44 et 45 exercent un effet de contention très faible, inférieure à 7 mmHg. La manche 41, également désignée par le terme « manchon », comprend trois éléments d'ancrage sous forme de rubans élastiques, à ses extrémités 46 et 48 et au niveau de l'articulation du coude 47. Ces éléments 46, 47 et 48 permettent de maintenir le manchon sur le bras. La manche permet d'augmenter le retour veineux, diminuer les picotements et la sensation de « bras lourds ».

Le mode opératoire décrit ci-après permet de mesurer la pression en mmHg exercée par un article d'habillement sur une zone du corps donnée à l'aide du capteur de pression du type KIKUHIME. Cette méthode, citée à titre non limitatif, a été utilisée pour la mesure des pressions exercées par les premières, secondes et troisièmes pièces formant les articles d'habillements selon la présente invention.

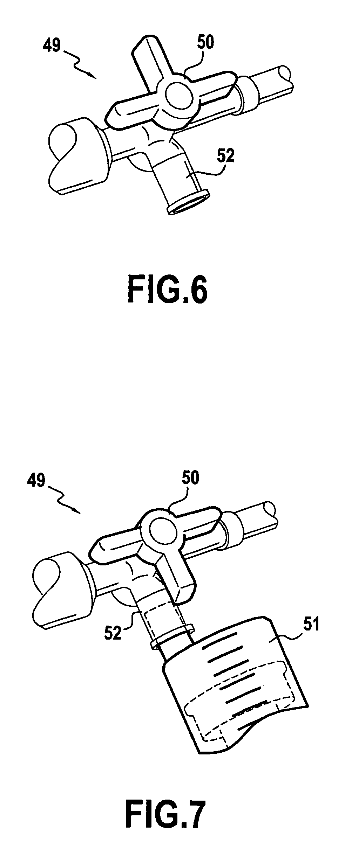

Une première étape de calibration de l'appareil capteur de pression KIKUHIME 49, représenté aux figures 6 et 7, est nécessaire avant d'effectuer les mesures de pression, comprenant notamment les étapes suivantes :
1) connecter la poche à air à la centrale,
2) ouvrir le robinet 50,
3) connecter la seringue 51 au robinet 52,
4) pousser ou tirer sur la seringue 51 jusqu'à ce que la valeur affichée par le capteur indique « O »,
5) refermer la voie du robinet 52 dans laquelle est connectée la seringue 51,
6) retirer la seringue 51,
7) le capteur est calibré.

La seconde étape est la mesure de la pression exercée par un article d'habillement sur une zone donnée du corps, comprenant notamment les étapes suivantes :
1) effectuer une marque sur la peau à l'endroit de la mesure,
2) le sujet revêt l'article d'habillement à tester, lequel article est alors dans sa position initiale,
3) remonter ledit article sur le membre ou le torse de façon à visualiser ladite marque,
4) placer la capteur sur ladite marque,
5) replacer l'article d'habillement dans sa position initiale,
6) vérifier que le capteur n'est pas glissé ou ne se soit pas retourné,
7) placer le sujet dans une position de référence, sans bouger, et plus particulièrement debout pour les mesures effectuées sur les articles d'habillement selon la présente invention,
8) relever la pression affichée sur le capteur (NB : la tolérance de l'appareil de mesure 49 est de +/- 1 mmHg d'après le fabricant),
9) effectuer deux fois cette manipulation pour s'assurer de la reproductibilité de la mesure.

## Revendications

1. Article d'habillement (1, 16, 28, 35, 41) à effet de contention localisé, pour la pratique d'un sport, obtenu par l'assemblage de premières pièces à élasticité de base et de secondes pièces à effet de contention, **caractérisé en ce que** :
- il comporte plusieurs types de secondes pièces aptes à produire des effets de contention différents, chaque type dans un intervalle de contention déterminé,
- chaque seconde pièce est agencée en sorte de produire un effet de contention soit longitudinale soit transversale,
- les secondes pièces des différents types sont agencées dans l'assemblage en sorte d'entourer totalement ou partiellement certaines sections transversales d'au moins un membre et/ou du torse et en sorte d'exercer un effet hétérogène de contention avec un gradient de pression décroissant vers le coeur.

2. Article selon la revendication 1 **caractérisé en ce qu'**il comporte des secondes pièces choisies parmi trois types, dont les intervalles de contention correspondent à des effets de contention respectivement faible, moyen et élevé.

3. Article selon la revendication 2 **caractérisé en ce que** le premier type à effet de contention faible correspond à un intervalle de pression de [10 ; 15] mm Hg, le deuxième type à effet de contention moyen à un intervalle de pression de [15 ; 20] mm Hg et le troisième type à effet de contention élevé à un intervalle de pression de [20 ; 25] mm Hg.

4. Article d'habillement selon l'une quelconques des revendications 1 à 3, **caractérisé en ce qu'**il se présente sous la forme d'une manche seule comprenant une partie d'avant-bras et une partie de bras.

5. Article d'habillement selon la revendication 4, **caractérisé en ce que** la partie d'avant-bras comprend une seconde pièce (5, 18, 29, 43) à effet de contention transversale recouvrant au moins les muscles fléchisseurs des doigts.

6. Article d'habillement selon la revendication 5, **caractérisé en ce que** ladite seconde pièce (5, 18, 29, 43) à effet de contention transversale exerce une pression sur les muscles fléchisseurs des doigts comprise dans un intervalle à effet de contention moyen, de préférence dans l'intervalle [15 ; 20] mmHg.

7. Article d'habillement selon l'une quelconque des revendications 4 à 6, **caractérisé en ce que** la partie de bras comprend une seconde pièce (3, 19, 30, 42) à effet de contention transversale recouvrant au moins les muscles biceps et triceps brachii.

8. Article d'habillement selon la revendication 7, **caractérisé en ce que** ladite seconde pièce (3, 19, 30, 42) à effet de contention transversale exerce une pression sur les muscles biceps et triceps brachii comprise dans un intervalle à effet de contention faible, et de préférence dans l'intervalle [10 ; 15] mmHg.

9. Article d'habillement selon l'une quelconque des revendications 4 à 8, **caractérisé en ce qu'**il comprend des éléments d'ancrage (46, 47, 48) à ses extrémités et au niveau de l'articulation du coude.

10. Article d'habillement selon l'une quelconque des revendications 1 à 3, **caractérisé en ce qu'**il présente sous la forme d'un maillot à manches longues (16, 28) comprenant une partie d'avant-bras, une partie de bras, une partie de poitrine, une partie d'épaule, une partie de dos et une partie d'abdomen.

11. Article d'habillement selon la revendication 10, **caractérisé en ce que** la partie d'épaule comprend une première pièce à élasticité de base (12, 23, 34) recouvrant le muscle deltoïde et la scapula.

12. Article d'habillement selon l'une ou l'autre des revendications 10 et 11, **caractérisé en ce que** la partie d'abdomen comprend une première pièce (11, 21, 33) à élasticité de base recouvrant les muscles abdominaux.

13. Article d'habillement selon l'une quelconque des revendications 10 à 12, **caractérisé en ce que** la partie de poitrine comprend une seconde pièce (2, 17) à effet de contention transversale recouvrant le muscle du grand pectoral.

14. Article d'habillement selon l'une quelconque des revendications 10 à 13, **caractérisé en ce que** la partie de dos comprend une seconde pièce à effet de contention transversale (24a) recouvrant le muscle du grand dorsal.

15. Article d'habillement selon la revendication 14, **caractérisé en ce que** la dite seconde pièce (24a) à effet de contention transversale se prolonge par une seconde pièce (24b) à effet de contention longitudinale sur le haut du dos qui se partage de part et d'autre de la nuque pour recouvrir notamment les muscles trapèzes et rhomboïdes.

16. Article d'habillement selon l'une quelconque des revendications 10 à 15, **caractérisé en ce que** la partie d'abdomen comprend une seconde pièce (25) à effet de contention transversale recouvrant le muscle grand oblique et le bas du muscle grand droit.

17. Article d'habillement selon l'une quelconque des revendications 10 à 16, **caractérisé en ce que** les secondes pièces (2, 17, 24a, 24b, 25) recouvrant les muscles du grand pectoral, du grand dorsal, du grand oblique, du grand droit, des trapèzes et rhomboïdes exercent une pression comprise dans un intervalle à effet de contention faible, et de préférence dans l'intervalle [10 ; 15] mmHg.

18. Article d'habillement selon l'une quelconque des revendications 10 à 17, **caractérisé en ce que** la partie d'avant-bras comprend une seconde pièce (5, 18, 29, 43) à effet de contention transversale recouvrant les muscles fléchisseurs des doigts exerçant une pression comprise dans un intervalle à effet de contention élevé, et de préférence dans l'intervalle [20 ; 25] mmHg.

19. Article d'habillement selon l'une quelconque des revendications 10 à 18, **caractérisé en ce que** la partie de bras comprend une seconde pièce (3, 19, 30, 42) à effet de contention transversale recouvrant les muscles biceps et triceps brachii exerçant une pression comprise dans un intervalle à effet de contention faible, et de préférence dans l'intervalle [10 ; 15] mmHg.

20. Article d'habillement selon l'une quelconque des revendications 10 à 19, **caractérisé en ce que** la seconde pièce (24a) à effet de contention transversale sur la partie de dos comprend une fausse couture (27) afin de plaquer ledit article sur le dos de l'usager, de préférence longeant la colonne vertébrale.

21. Article d'habillement selon l'une quelconque des revendications 10 à 20, **caractérisé en ce qu'**il comporte des troisièmes pièces (14, 15, 20, 32) destinées à recouvrir les aisselles se présentant sous la forme de filet pour favoriser l'évacuation de la chaleur et de la transpiration.

22. Article d'habillement selon l'une quelconque des revendications 1 à 3 **caractérisé en ce qu'**il se présente sous la forme d'un collant (35) comprenant une partie antérieure de la cuisse, une partie postérieure de la cuisse, une partie de genou et une partie de jambe.

23. Article d'habillement selon la revendication 22, **caractérisé en ce que** la partie antérieure de la cuisse comprend une seconde pièce (7, 38) à effet de contention transversale recouvrant le muscle droit, le muscle du vaste externe, et les muscles adducteurs ou du vaste interne.

24. Article d'habillement selon l'une ou l'autre des revendications 22 et 23, **caractérisé en ce que** la partie postérieure de la cuisse comprend une seconde pièce (7, 38) à effet de contention transversale recouvrant les muscles biceps fémoral et les muscles adducteurs ou les muscles semi-membraneux.

25. Article d'habillement selon l'une quelconque des revendications 22 à 24, **caractérisé en ce que** la partie de genou comprend une seconde pièce à effet de contention transversale (7, 38) recouvrant sur sa face antérieure la tête de la fibula et sur sa face postérieure l'intérieur de l'articulation du genou pour se prolonger sur la partie haute du gastrocnémien en passant sous ladite articulation du genou.

26. Article d'habillement selon l'une quelconque des revendications 22 à 25, **caractérisé en ce que** lesdites secondes pièces (7, 38) à effet de contention soit transversale soit longitudinale exercent une pression comprise dans un intervalle à effet de contention faible, et de préférence dans l'intervalle [10 ; 15] mmHg.

27. Article d'habillement selon l'une quelconque des revendications 22 à 26, **caractérisé en ce que** la partie de jambe comprend une seconde pièce (9, 40) à effet de contention transversale recouvrant les muscles gastrocnémiens et les muscles extenseurs des orteils.

28. Article d'habillement selon la revendication 27, **caractérisé en ce que** ladite seconde pièce (9, 40) à effet de contention transversale exercent une pression comprise dans un intervalle à effet de contention élevé, et de préférence dans l'intervalle [20 ; 25] mmHg.

29. Article d'habillement selon l'une quelconque des revendications 1 à 3, **caractérisé en ce qu'**il se présente sous la forme d'une combinaison à manches longues (1), notamment pour la natation, comprenant une partie d'avant-bras, une partie de bras, une partie de poitrine, une partie d'épaule, une partie de dos, une partie d'abdomen, une partie de fessier, une partie antérieure de la cuisse, une partie postérieure de la cuisse, une partie de genou et une partie de jambe selon l'une quelconque des revendications 11 à 21 et des revendications 23 à 28.

30. Article d'habillement selon la revendication 29, **caractérisé en ce que** la partie de dos comprend une seconde pièce à effet de contention longitudinale (6) recouvrant les muscles érecteurs du rachis et se prolongeant vers le bas du dos sur la partie basse du muscle du grand fessier et le muscle du tenseur fascia lata, et vers le haut du dos longe la colonne vertébrale pour se partager de part et d'autre de la nuque et recouvrir notamment les muscles trapèzes et rhomboïdes, et **en ce que** ladite seconde pièce exerce une pression comprise dans un intervalle à effet de contention faible, et de préférence dans l'intervalle [10 ; 15] mmHg.

31. Article d'habillement selon l'une ou l'autre des revendications 29 et 30, **caractérisé en ce que** la partie d'abdomen comprend une seconde pièce (6) à effet de contention longitudinale recouvrant, en passant sous le nombril de part et d'autre de la taille, les muscles du petit oblique et du grand droit, et **en ce que** ladite seconde pièce exerce une pression comprise dans un intervalle à effet de contention faible, et de préférence dans l'intervalle [10 ; 15] mmHg.

32. Article d'habillement selon l'une ou l'autre des revendications 4, 10 et 29, **caractérisé en ce qu'**il comporte une première pièce à élasticité de base recouvrant l'articulation du coude, et **en ce que** ladite première pièce est préformée selon un angle de 45° au niveau de l'articulation du coude correspondant à l'angle de flexion naturel du coude.

33. Utilisation d'un article d'habillement selon la revendication 4 pour l'escalade ou la randonnée en montagne.

34. Utilisation d'un article d'habillement selon l'une ou l'autre des revendications 10, 22 et 29 pour la natation ou le surf.

## Claims

1. A garment (1, 16, 28, 35, 41) with localized compression effect for performing a sport, the garment being obtained by assembling first pieces of basic elasticity and second pieces that present a compression effect, the garment being **characterized in that**:
· it comprises a plurality of types of second piece suitable for producing different compression effects, each type acting in a determined compression range;
· each second piece is arranged so as to produce a compression effect that is either longitudinal or transverse; and
· the second pieces of the various types are arranged in the assembly so as to surround certain transverse sections of at least one limb and/or the torso in full or in part and so as to exert a heterogeneous compression effect with a pressure gradient that decreases towards the heart.

2. A garment according to claim 1, **characterized in that** it includes second pieces selected from three types, having compression ranges that correspond respectively to weak, medium, and strong compression.

3. A garment according to claim 2, **characterized in that** the first type of weak compression effect corresponds to a pressure range of [10, 15] mmHg, the second type of medium compression effect corresponds to a pressure range of [15, 20] mmHg, and the third type of strong compression effect corresponds to a pressure range of [20, 25] mmHg.

4. A garment according to any one of claims 1 to 3, **characterized in that** it is in the form of a sleeve on its own, comprising a forearm portion and an upper arm portion.

5. A garment according to claim 4, **characterized in that** the forearm portion comprises a second piece (5, 18, 29, 43) of transverse compression effect covering at least the finger flexor muscles.

6. A garment according to claim 5, **characterized in that** said second piece (5, 18, 29, 43) of transverse compression effect exerts pressure on the finger flexor muscles lying in a medium compression effect range, preferably in the range [15, 20] mmHg.

7. A garment according to any one of claims 4 to 6, **characterized in that** the upper arm portion includes a second piece (3, 19, 30, 42) of transverse compression effect covering at least the biceps and triceps brachii muscles.

8. A garment according to claim 7, **characterized in that** said second piece (3, 19, 30, 42) of transverse compression effect exerts pressure on the biceps and triceps brachii muscles lying in a weak compression effect range, and preferably in the range [10, 15] mmHg.

9. A garment according to any one of claims 4 to 8, **characterized in that** it includes anchoring elements (46, 47, 48) at its ends and at the elbow joint.

10. A garment according to any one of claims 1 to 3, **characterized in that** it is in the form of a long-sleeved top (16, 28) having a forearm portion, an upper arm portion, a chest portion, a shoulder portion, a back portion, and an abdomen portion.

11. A garment according to claim 10, **characterized in that** the shoulder portion includes a first piece (12, 23, 34) of basic elasticity covering the deltoid muscle and the shoulder blade.

12. A garment according to claim 10 or claim 11, **characterized in that** the abdomen portion comprises a first piece (11, 21, 33) of basic elasticity covering the abdominal muscles.

13. A garment according to any one of claims 10 to 12, **characterized in that** the chest portion includes a second piece (2, 17) of transverse compression effect covering the greater pectoral muscle.

14. A garment according to any one of claims 10 to 13, **characterized in that** the back portion includes a second piece (24a) of transverse compression effect covering the broad muscle of the back.

15. A garment according to claim 14, **characterized in that** said second piece (24a) of transverse compression effect is extended by a second piece (24b) of longitudinal compression effect over the top of the back, which piece is shared between opposite sides of the nape of the neck, in particular to cover the trapezius and rhomboid muscles.

16. A garment according to any one of claims 10 to 15, **characterized in that** the abdominal portion includes a second piece (25) of transverse compression effect covering the external oblique muscle and the bottom of the rectus muscle.

17. A garment according to any one of claims 10 to 16, **characterized in that** the second pieces (2, 17, 24a, 24b, 25) covering the greater pectoral muscle, the broad muscle of the back, the external oblique muscle, the rectus muscle, the trapezius muscles, and the rhomboid muscles, exert pressure lying in a weak compression effect range, preferably in the range [10, 15] mmHg.

18. A garment according to any one of claims 10 to 17, **characterized in that** the forearm portion includes a second piece (5, 18, 29, 43) of transverse compression effect covering the finger flexor muscles and exerting pressure lying in a strong compression effect range, preferably in the range [20, 25] mmHg.

19. A garment according to any one of claims 10 to 18, **characterized in that** the upper arm portion includes a second piece (3, 19, 30, 42) of transverse compression effect covering the biceps and triceps brachii muscles and exerting pressure lying in a weak compression effect range, and preferably in the range [10, 15] mmHg.

20. A garment according to any one of claims 10 to 19, **characterized in that** the second piece (24a) of transverse compression effect on the back portion includes a mock seam (27) so as to press said garment on the user's back, preferably along the spinal column.

21. A garment according to any one of claims 10 to 20, **characterized in that** it includes third pieces (14, 15, 20, 32) for covering the armpits and in the form of a net to encourage evacuating heat and perspiration.

22. A garment according to any one of claims 1 to 3, **characterized in that** it is in the form of tights (35) comprising an anterior thigh portion, a posterior thigh portion, a knee portion, and a leg portion.

23. A garment according to claim 22, **characterized in that** the anterior thigh portion includes a second piece (7, 38) of transverse compression effect covering the rectus muscle, the lateral vastus muscle, and the adductor muscles or the medial vastus muscle.

24. A garment according to claim 22 or claim 23, **characterized in that** the posterior portion of the thigh includes a second piece (7, 38) of transverse compression effect covering the femoral biceps muscles and the adductor muscles or the semi-membraneous muscles.

25. A garment according to any one of claims 22 to 24, **characterized in that** the knee portion includes a second piece (7, 38) of transverse compression effect covering with its anterior face the head of the fibula and with its posterior face the inside of the knee joint so as to extend over the top portion of the gastrocnemius, passing under said knee joint.

26. A garment according to any one of claims 22 to 25, **characterized in that** said second pieces (7, 38) of longitudinal or transverse compression effect exert pressure lying in a weak pressure effect range, and preferably in the range [10, 15] mmHg.

27. A garment according to any one of claims 22 to 26, **characterized in that** the leg portion includes a second piece (9, 40) of transverse compression effect covering the gastrocnemius muscles and the toe extensor muscles.

28. A garment according to claim 27, **characterized in that** said second piece (9, 40) of transverse compression effect exerts pressure lying in a strong compression effect range, and preferably in the range [20, 25] mmHg.

29. A garment according to any one of claims 1 to 3, **characterized in that** it is in the form of a long-sleeved costume (1), in particular for swimming, comprising a forearm portion, an upper arm portion, a chest portion, a shoulder portion, a back portion, an abdomen portion, a buttocks portion, a thigh anterior portion, a thigh posterior portion, a knee portion, and a leg portion according to any one of claims 11 to 21 and claims 23 to 28.

30. A garment according to claim 29, **characterized in that** the back portion includes a second piece (6) of longitudinal compression effect covering the erector muscles of the spine and extending towards the bottom of the back over the low portion of the luteal muscle and the tensor muscle of the fascia lata, and towards the top of the back along the spinal column to be shared on either side of the nape of the neck and cover in particular the trapezius and rhomboid muscles, and **in that** said second piece exerts pressure lying in a weak compression effect range, and preferably in the range [10, 15] mmHg.

31. A garment according to claim 29 or claim 30, **characterized in that** the abdomen portion includes a second piece (6) of longitudinal compression effect covering, on passing below the navel on either side of the waist, the internal oblique muscle and the rectus muscle, and **in that** the second piece exerts pressure lying in a weak compression effect range, and preferably in the range [10, 15] mmHg.

32. A garment according to any one of claims 4, 10, and 29, **characterized in that** it includes a first piece of basic elasticity covering the elbow joint, and **in that** said first piece is preshaped with an angle of 45° at the elbow joint, corresponding to the natural bending angle of the elbow.

33. The use of a garment according to claim 4 for climbing or mountain trekking.

34. The use of a garment according to any one of claims 10, 22, and 29, for swimming or surfing.

## Patentansprüche

1. Bekleidungsartikel (1, 16, 28, 35, 41) mit lokaler Kompressionswirkung, zur Ausübung eines Sports, der durch Verbinden von eine Grundelastizität aufweisenden ersten Teilen und von eine Kompressionswirkung aufweisenden zweiten Teilen erhalten wird, **dadurch gekennzeichnet, daß:**
- er mehrere Arten von zweiten Teilen umfaßt, die geeignet sind, unterschiedliche Kompressionswirkungen zu erzeugen, jede Art in einem bestimmten Kompressionsbereich,
- jedes zweite Teil derart angeordnet ist, daß es eine entweder längsgerichtete oder quergerichtete Kompressionswirkung erzeugt,
- die zweiten Teile der unterschiedlichen Arten in der Anordnung derart angeordnet sind, daß sie bestimmte Querschnitte wenigstens eines Gliedes und/oder des Rumpfes vollständig oder teilweise umgeben und daß sie eine heterogene Kompressionswirkung mit einem zum Herzen hin abnehmenden Druckgradienten ausüben.

2. Artikel nach Anspruch 1, **dadurch gekennzeichnet, daß** er zweite Teile umfaßt, die aus drei Arten ausgewählt sind, deren Kompressionsbereiche einer geringen, einer mittleren bzw. einer hohen Kompressionswirkung entsprechen.

3. Artikel nach Anspruch 2, **dadurch gekennzeichnet, daß** die erste Art mit geringer Kompressionswirkung einem Druckbereich von [10; 15] mmHg, die zweite Art mit mittlerer Kompressionswirkung einem Druckbereich von [15; 20] mmHg und die dritte Art mit hoher Kompressionswirkung einem Druckbereich von [20; 25] mmHg entspricht.

4. Bekleidungsartikel nach einem der Ansprüche 1 bis 3, **dadurch gekennzeichnet, daß** er in Form eines einzelnen Ärmels mit einem Unterarmteil und einem Oberarmteil vorliegt.

5. Bekleidungsartikel nach Anspruch 4, **dadurch gekennzeichnet, daß** der Unterarmteil ein zweites Teil (5, 18, 29, 43) mit quergerichteter Kompressionswirkung umfaßt, das wenigstens die Fingerbeuger bedeckt.

6. Bekleidungsartikel nach Anspruch 5, **dadurch gekennzeichnet, daß** das zweite Teil (5, 18, 29, 43) mit quergerichteter Kompressionswirkung einen Druck auf die Fingerbeuger ausübt, der in einem Bereich mit mittlerer Kompressionswirkung, vorzugsweise im Bereich [15; 20] mmHg liegt.

7. Bekleidungsartikel nach einem der Ansprüche 4 bis 6, **dadurch gekennzeichnet, daß** der Oberarmteil ein zweites Teil (3, 19, 30, 42) mit quergerichteter Kompressionswirkung umfaßt, das wenigstens den Biceps und den Triceps brachii bedeckt.

8. Bekleidungsartikel nach Anspruch 7, **dadurch gekennzeichnet, daß** das zweite Teil (3, 19, 30, 42) mit quergerichteter Kompressionswirkung einen Druck auf den Biceps und den Triceps brachii ausübt, der in einem Bereich mit geringer Kompressionswirkung und vorzugsweise im Bereich [10; 15] mmHg liegt.

9. Bekleidungsartikel nach einem der Ansprüche 4 bis 8, **dadurch gekennzeichnet, daß** er an seinen Enden und im Bereich des Ellenbogengelenks Verankerungselemente (46, 47, 48) umfaßt.

10. Bekleidungsartikel nach einem der Ansprüche 1 bis 3, **dadurch gekennzeichnet, daß** er in Form eines Langarmtrikots (16, 28) mit einem Unterarmteil, einem Oberarmteil, einem Brustteil, einem Schulterteil, einem Rückenteil und einem Bauchteil vorliegt.

11. Bekleidungsartikel nach Anspruch 10, **dadurch gekennzeichnet, daß** der Schulterteil ein erstes Teil mit Grundelastizität (12, 23, 34) umfaßt, das den Deltamuskel und das Schulterblatt bedeckt.

12. Bekleidungsartikel nach dem einen oder anderen der Ansprüche 10 und 11, **dadurch gekennzeichnet, daß** der Bauchteil ein erstes Teil (11, 21, 33) mit Grundelastizität umfaßt, das die Bauchmuskeln bedeckt.

13. Bekleidungsartikel nach einem der Ansprüche 10 bis 12, **dadurch gekennzeichnet, daß** der Brustteil ein zweites Teil (2, 17) mit quergerichteter Kompressionswirkung umfaßt, das den großen Brustmuskel bedeckt.

14. Bekleidungsartikel nach einem der Ansprüche 10 bis 13, **dadurch gekennzeichnet, daß** der Rückenteil ein zweites Teil mit quergerichteter Kompressionswirkung (24a) umfaßt, das den großen Rückenmuskel bedeckt.

15. Bekleidungsartikel nach Anspruch 14, **dadurch gekennzeichnet, daß** das zweite Teil (24a) mit quergerichteter Kompressionswirkung durch ein zweites Teil (24b) mit längsgerichteter Kompressionswirkung am oberen Teil des Rückens fortgesetzt ist, das sich auf beiden Seiten des Nackens teilt, um insbesondere die Trapez- und Rautenmuskeln zu bedecken.

16. Bekleidungsartikel nach einem der Ansprüche 10 bis 15, **dadurch gekennzeichnet, daß** der Bauchteil ein zweites Teil (25) mit quergerichteter Kompressionswirkung umfaßt, das den großen schrägen Muskel sowie den unteren Teil des großen geraden Muskels bedeckt.

17. Bekleidungsartikel nach einem der Ansprüche 10 bis 16, **dadurch gekennzeichnet, daß** die zweiten Teile (2, 17, 24a, 24b, 25), die den großen Brustmuskel, den großen Rückenmuskel, den großen schrägen Muskel, den großen geraden Muskel, die Trapez- und Rautenmuskeln bedecken, einen Druck ausüben, der in einem Bereich mit geringer Kompressionswirkung und vorzugsweise im Bereich [10; 15] mmHg liegt.

18. Bekleidungsartikel nach einem der Ansprüche 10 bis 17, **dadurch gekennzeichnet, daß** der Unterarmteil ein die Fingerbeuger bedeckendes zweites Teil (5, 18, 29, 43) mit quergerichteter Kompressionswirkung umfaßt, das einen Druck in einem Bereich mit hoher Kompressionswirkung und vorzugsweise im Bereich [20; 25] mmHg ausübt.

19. Bekleidungsartikel nach einem der Ansprüche 10 bis 18, **dadurch gekennzeichnet, daß** der Oberarmteil ein den Biceps und den Triceps brachii bedeckendes zweites Teil (3, 19, 30, 42) mit quergerichteter Kompressionswirkung umfaßt, das einen Druck in einem Bereich mit geringer Kompressionswirkung und vorzugsweise im Bereich [10; 15] mmHg ausübt.

20. Bekleidungsartikel nach einem der Ansprüche 10 bis 19, **dadurch gekennzeichnet, daß** das zweite Teil (24a) mit quergerichteter Kompressionswirkung am Rückenteil eine imitierte Naht (27) aufweist, um den Artikel an den Rücken des Benutzers, vorzugsweise entlang der Wirbelsäule, zu drücken.

21. Bekleidungsartikel nach einem der Ansprüche 10 bis 20, **dadurch gekennzeichnet, daß** er dritte Teile (14, 15, 20, 32) zum Bedecken der Achseln umfaßt, die in Form eines Netzes vorliegen, um das Abführen der Wärme und des Schweißes zu begünstigen.

22. Bekleidungsartikel nach einem der Ansprüche 1 bis 3, **dadurch gekennzeichnet, daß** er in Form einer Strumpfhose (35) mit einem vorderseitigen Oberschenkelteil, einem rückseitigen Oberschenkelteil, einem Knieteil und einem Unterschenkelteil vorliegt.

23. Bekleidungsartikel nach Anspruch 22, **dadurch gekennzeichnet, daß** der vorderseitige Oberschenkelteil ein zweites Teil (7, 38) mit quergerichteter Kompressionswirkung umfaßt, das den geraden Muskel, den Vastus externus und die Adduktoren-Muskeln oder den Vastus internus bedeckt.

24. Bekleidungsartikel nach dem einen oder anderen der Ansprüche 22 und 23, **dadurch gekennzeichnet, daß** der rückseitige Oberschenkelteil ein zweites Teil (7, 38) mit quergerichteter Kompressionswirkung umfaßt, das den Biceps femoris und die Adduktoren-Muskeln oder die halbmembranösen Muskeln bedeckt.

25. Bekleidungsartikel nach einem der Ansprüche 22 bis 24, **dadurch gekennzeichnet, daß** der Knieteil ein zweites Teil mit quergerichteter Kompressionswirkung (7, 38) umfaßt, das an seiner Vorderseite den Wadenbeinkopf und an seiner Rückseite die Innenseite des Kniegelenks bedeckt, um sich unter dem Kniegelenk verlaufend am oberen Teil des Wadenmuskels fortzusetzen.

26. Bekleidungsartikel nach einem der Ansprüche 22 bis 25, **dadurch gekennzeichnet, daß** die zweiten Teile (7, 38) mit entweder quergerichteter oder längsgerichteter Kompressionswirkung einen Druck in einem Bereich mit geringer, Kompressionswirkung und vorzugsweise im Bereich [10; 15] mmHg ausüben.

27. Bekleidungsartikel nach einem der Ansprüche 22 bis 26, **dadurch gekennzeichnet, daß** der Beinteil ein zweites Teil (9, 40) mit quergerichteter Kompressionswirkung umfaßt, das die Wadenmuskeln und die Zehenstrecker bedeckt.

28. Bekleidungsartikel nach Anspruch 27, **dadurch gekennzeichnet, daß** das zweite Teil (9, 40) mit quergerichteter Kompressionswirkung einen Druck in einem Bereich mit hoher Kompressionswirkung und vorzugsweise im Bereich [20; 25] mmHg ausübt.

29. Bekleidungsartikel nach einem der Ansprüche 1 bis 3, **dadurch gekennzeichnet, daß** er in Form eines Langarmanzugs (1), insbesondere zum Schwimmen, umfassend einen Unterarmteil, einen Oberarmteil, einen Brustteil, einen Schulterteil, einen Rückenteil, einen Bauchteil, einen Gesäßteil, einen vorderseitigen Oberschenkelteil, einen rückseitigen Oberschenkelteil, einen Knieteil und einen Unterschenkeiteil nach einem der Ansprüche 11 bis 21 und der Ansprüche 23 bis 28 vorliegt.

30. Bekleidungsartikel nach Anspruch 29, **dadurch gekennzeichnet, daß** der Rückenteil ein zweites Teil mit längsgerichteter Kompressionswirkung (6) umfaßt, das den Musculus erector spinae bedeckt und sich zum unteren Bereich des Rückens über den unteren Teil des großen Gesäßmuskels und den Musculus Tensor fascia latae sowie zum oberen Bereich des Rückens entlang der Wirkbelsäule fortsetzt, um sich auf beiden Seiten des Nackens zu teilen und insbesondere die Trapez- und Rautenmuskeln zu bedecken, und daß das zweite Teil einen Druck in einem Bereich mit geringer Kompressionswirkung und vorzugsweise im Bereich [10; 15] mmHg ausübt.

31. Bekleidungsartikel nach dem einen oder anderen der Ansprüche 29 und 30, **dadurch gekennzeichnet, daß** der Bauchteil ein zweites Teil (6) mit längsgerichteter Kompressionswirkung umfaßt, das unter dem Nabel auf beiden Seiten der Taille verlaufend die kleinen schrägen und großen geraden Muskeln bedeckt, und daß das zweite Teil einen Druck in einem Bereich mit geringer Kompressionswirkung und vorzugsweise im Bereich [10; 15] mmHg ausübt.

32. Bekleidungsartikel nach dem einen oder anderen der Ansprüche 4, 10 und 29, **dadurch gekennzeichnet, daß** er ein erstes Teil mit Grundelastizität umfaßt, das das Ellenbogengelenk bedeckt, und daß das erste Teil im Bereich des Ellenbogengelenks in einem Winkel von 45°, der dem natürlichen Beugewinkel des Ellenbogens entspricht, vorgeformt ist.

33. Verwendung eines Bekleidungsartikels nach Anspruch 4 zum Bergsteigen oder für Bergwandertouren.

34. Verwendung eines Bekleidungsartikels nach dem einen oder anderen der Ansprüche t0, 22 und 29 zum Schwimmen oder Surfen.
